# EUROPEAN PATENT APPLICATION

(11) **EP 2 408 217 A2**
(43) Date of publication of application: **18.01.2012**
(21) Application number: 11460036.4
(22) Date of filing: 09.07.2011
(51) Int. Cl.: H04N 13/04, G02B 27/01

(54) **Method of virtual 3d image presentation and apparatus for virtual 3d image presentation**

(30) Priority: 12.07.2010 PL 39180010
(71) Applicant: DiagNova Technologies Spólka Cywilna Marcin Pawel Just, Michal Hugo Tyc, Monika Morawska-Kochman, 54-424 Wroclaw (PL)
(72) Inventor: Just, Marcin Pawel, 51-128 Wroclaw (PL); Tyc, Michal Hugo, 54-438 Wroclaw (PL)

(57) **Abstract**

The present invention relates to a method of virtual 3D image presentation and an apparatus for virtual 3D image presentation, intended especially for applications in virtual reality systems.

The method consist in that the optical power of the spectator's eye (3) is continuously measured with the eye optical power measurement system (1), and, based on the measured optical power, the sharp vision plane of the spectator's eye (3) is determined, and then the sharp vision plane of the spectator's eye (3) is superimposed onto the surface of the screen (7, 8) presenting the virtual 3D contents, and simultaneously, the depth of focus for the virtual 3D contents is determined, such that objects, laid in the virtual scene at their apparent distance from the spectator equal to the current unmodified sharp vision distance of his/her eye, are presented as sharp, while the objects laid closer or further away are presented with blur, where the amount of blur is a non-decreasing function of the absolute difference of the spectator's eye sharp vision distance and the apparent distance to the object in the virtual scene. The apparatus comprising of a microprocessor device (4) connected to at least one eye optical power measuring system (1), which is connected to at least one screen positioning mechanism (9) that dynamically positions the miniature screen (8), and the miniature screen (8), connected to the microprocessor device (4), is always positioned at the modified sharp vision distance, irrespective of the spectator's eye (3) accommodation, and furthermore, the static focusing system (10) placed between the miniature screen (8) and the spectator's eye (3).

## Description

### Field of the Invention

The present invention relates to a method of virtual 3D image presentation and an apparatus for virtual 3D image presentation, intended especially for applications in virtual reality systems.

### Background to the Invention

The known methods of generation of virtual 3D image with depth of focus not covering the entire 3D scene are limited to pre-determining, at the design stage of the presented virtual reality, the range of distances within which the displayed 3D image should be perceived by a spectator as sharp and full of detail. This significantly limits the spectator, in his/her role, to a passive viewer, just looking at the virtual 3D contents in the way dictated by the screenplay. Conversely, in the natural vision conditions, the depth of focus is dynamically adjusted by the spectator's brain, by means of eye accommodation mechanisms, to reflect current spectator's needs and varying environmental conditions.

Early methods of 3D virtual image presentation ignored the depth of focus aspect by displaying on a screen an image which was entirely sharp, independent of the apparent distances of virtual objects from the spectator's eye. In such a case, the actual distance of sharp vision of the spectator's eye is constant and coincides with the distance of the screen plane. Due to the presented image being sharp independent of the apparent distance, the spectator does not need to accommodate, and prolonged viewing of such an image does not cause negative sensations manifesting by fatigue and/or headache. However, such an image does not provide the spectator with the full of natural experience.

Newer software applications presenting three-dimensional virtual reality, including computer games, apply the Depth of Field technique to improve the degree of realism in their generated scene, however, this may entail discrepancy between the sharp vision distance resulting from eye accommodation, which must be fixed at the screen displaying the contents, and the apparent distance of sharp vision, resulting from the depth of focus applied by the screenwriter. The problem becomes particularly important when the 3D contents is presented by means of stereoscopic devices, such as polarization filter glasses, color filter (anaglyphic) glasses, shutter glasses, and helmets or goggles displaying separate contents for each eye. In such a case, the discrepancy between eye's actual sharp vision distance and the apparent distance dictated by the depth of focus in the generated 3D image may much more frequently lead to discomfort and other negative sensations.

A method of and system for projecting three-dimensional movie pictures known from patent application No. W09107696 (also filed as PL287879) consists in alternately projecting corresponding left-eye and right-eye images onto a dome-shaped projection screen from two separate film strips and through separate wide-angle lenses. The lenses are positioned close together with their projection axes in a common vertical plane to achieve lateral co-incidence of the projected images and oriented in that plane to achieve vertical co-incidence of the images. Each person viewing the motion picture is provided with glasses that have lenses in the form of liquid crystal cells arranged to alternately block the left and right eyes of the person in synchronism with the projection of right-eye and left-eye images respectively, so that a stereoscopic effect is perceived.

Method and system for the presentation of simple stereoscopic images, particularly for testing vision, known from patent application No. PL379400, consists in simultaneous displaying the left and right half-images on a single graphical screen, while the left and right half-image parts are separated from the single image presented on the screen by means of a matrix polarization switch. The system of stereoscopic image presentation comprises in passive polarization glasses having one polarization filter for each eye, positioned such that the polarization angles for left and right eye are mutually orthogonal, and the matrix polarization switch is realized in a form of monochromatic LCD panel consisting of individually controlled cells which can either rotate the polarization plane of the incident light by 90 degrees or pass the incident light without changing its polarization state, and the cells are very quickly switched between both states.

A method of and apparatus for generating three-dimensional images, known from patent application No. W09417638 (also filed as PL309947), consists in displaying an imagery including two or more spaced-apart angles of view about a common centre and being segmented and separated. The segmented and separated imagery is viewed through grid means of a size and shape corresponding substantially to dimensions of the segments of imagery. The grid is placed at a distance from the screen such that segments of imagery acquired left of a common image centre are seen through the grid substantially by the left eye of a viewer, while segments of imagery acquired right of a common image centre are seen through the grid substantially by the right eye of a viewer. The relationship of left and right segments of imagery relative to said grid is maintained by oscillation of said grid relative to said segments of imagery appearing on said screen.

A method and apparatus for the production of three-dimensional images, known from patent application No. PL179677 (also filed as WO9746913), utilizes a multi-lens camera and a multi-lens enlarger configured according to a standard of arrangements. The number of lenses used in the camera and printer is selected to be greater than the resolution capabilities of the human eye and the lenticular print system. The width of a zone of the lineiform image is determined by the distance between two adjacent images on the focal plane of the lenticular screen (10) of a point projected from a distance at or beyond the distance limit through adjacent projecting apertures of the enlarger. The projecting (182, 186, 188) apertures of the enlarger are linearly arrayed and equally spaced within the unique accepting angle corresponding to the distance limit to construct a lineiform image without gaps between zones and without gaps between lines. Accordingly, a three-dimensional image having orthoscopic effect, and without stroboscopic effect, is produced in a one-step imaging and one-step composing process.

### Summary of the Invention

A method in accordance with the present invention consists in that the optical power of the spectator's eye is continuously measured with the eye optical power measurement system, and, based on the measured optical power, the sharp vision plane of the spectator's eye is determined, and then the sharp vision plane of the spectator's eye is superimposed onto the surface of the screen presenting the virtual 3D contents. Simultaneously, the depth of focus for the virtual 3D contents is determined, such that objects, laid in the virtual scene at their apparent distance from the spectator equal to the current unmodified sharp vision distance of his/her eye, are presented as sharp, while the objects laid closer or further away are presented with blur, where the amount of blur is a non-decreasing function of the absolute difference of the spectator's eye sharp vision distance and the apparent distance to the object in the virtual scene.

It is advantageous that the sharp vision plane of the spectator's eye is superimposed onto the surface of the screen presenting the virtual 3D contents with the help of the dynamic focusing system, which modifies the refracting power of the spectator's eye.

It is advantageous that the sharp vision plane of the spectator's eye is modified with the static focusing system, where the surface of the miniature screen is positioned with the help of the screen positioning mechanism and superimposed onto the eye's sharp vision plane.

The apparatus in accordance with the present invention comprises, in its first embodiment, a microprocessor device connected to at least one eye optical power measuring system, which is connected to at least one dynamic focusing system that dynamically modifies the optical power of the spectator's eye, and the free-standing screen, connected to the microprocessor device, which is always placed at the modified sharp vision distance, irrespective of the spectator's eye accommodation.

It is advantageous that the microprocessor device is connected to two eye optical power measurement systems, where the first one measures the optical power of the spectator's left eye, and the other measures the optical power of the spectator's right eye.

The apparatus in accordance with the present invention comprises, in its second embodiment, a microprocessor device connected to at least one eye optical power measuring system, which is connected to at least one screen positioning mechanism that dynamically positions the miniature screen, and the miniature screen, connected to the microprocessor device, which is always positioned at the modified sharp vision distance, irrespective of the spectator's eye accommodation, and furthermore, the static focusing system placed between the miniature screen and the spectator's eye.

It is advantageous that the microprocessor device is connected to two eye optical power measurement systems, where the first one measures the optical power of the spectator's left eye, and the other measures the optical power of the spectator's right eye.

As will be appreciated, in the method in accordance with the present invention, the depth of focus in the generated 3D image is dynamically controlled and its range is adjusted such that objects laid in the distance currently coinciding with the spectator's eye sharp vision distance resulting from eye accommodation, and the current sharp vision distance of the spectator's eye is determined continuously by measuring the optical parameters of the spectator's eye, including the optical power of the cornea-lens system. The experience realism of the spectator viewing a virtual 3D scene is significantly improved, and also the problem of discrepancy between the accommodation sharp vision distance of the spectator's eye, which is fixed at the screen displaying the 3D contents, and the apparent distance of virtual objects in the 3D scene, particularly the sharply displayed objects which define the virtual sharp vision range. The apparatus in accordance with the present invention is equipped with the optical-electronic system measuring the momentary refracting power of the optical system of one or both eyes, which is connected to the dynamic focusing system that compensates for the accommodation effects in both eyes, separately or jointly, and a microprocessor device generating the virtual reality 3D image.

The apparatus may be made in a form of goggles either independent of the device displaying the 3D contents, or including integrated screens displaying the 3D contents.

### Brief Description of the Drawings

An example of a method and an apparatus for performing the invention are now described with reference to the accompanying drawings, in which:

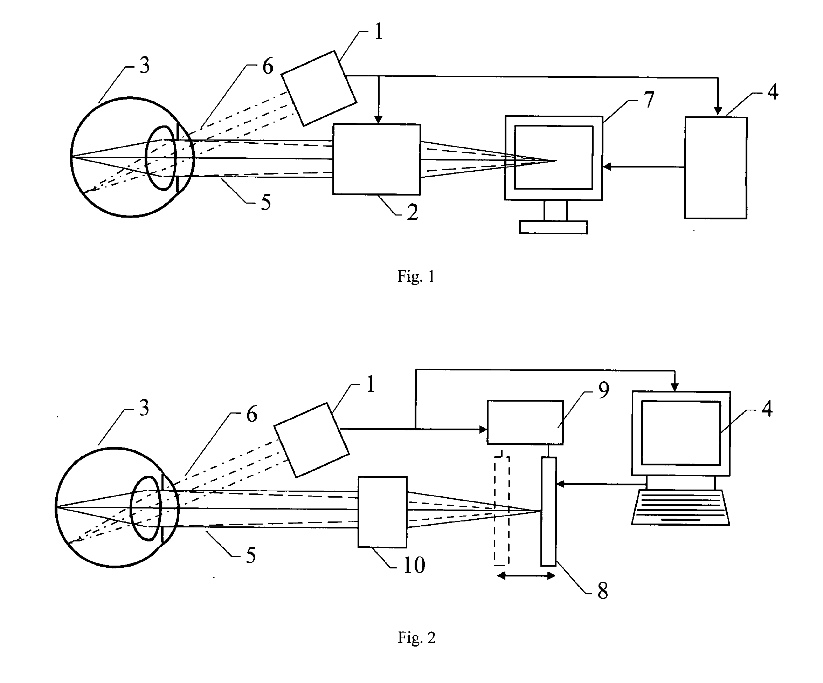

Figure 1 shows a general schematic representation of the first embodiment of the apparatus, equipped with the dynamic focusing system and the free-standing screen displaying the 3D contents;
Figure 2 shows a general schematic representation of the second embodiment of the apparatus, equipped with the built-in miniature screen and the static focusing system.

### Description of Examples

### Example 1

Method of virtual 3D image presentation consist in that the spectator's eye 3 optical power is continuously measured with the help of eye optical power measurement system 1 in which the measurement light beam 6 is generated and analyzed, and based on the measured optical power, the spectator's eye 3 sharp vision plane is determined, and then the spectator's eye 3 sharp vision plane is superimposed on the surface of the free-standing screen 7 presenting the virtual 3D contents and, simultaneously, the depth of focus for the virtual 3D contents is determined. The spectator's eye 3 sharp vision plane is superimposed on the surface of the free-standing screen 7 the virtual 3D contents with the help of dynamic focusing system 2 in which the eye's 3 refracting power is modified for the imaging light beam 5 projecting the virtual 3D contents from the free-standing screen 7 to the spectator's eye 3. Objects, laid in the virtual scene at their apparent distance from the spectator equal to the current unmodified sharp vision distance of his/her eye 3, are presented as sharp, while the objects laid closer or further away are presented with blur, where the amount of blur is a non-decreasing function of the absolute difference of the spectator's eye 3 sharp vision distance and the apparent distance to the object in the virtual scene.

### Example 2

Method of virtual 3D image presentation is carried out as in the Example 1 with the difference that the surface of the miniature screen 8 is positioned with the help of the screen positioning mechanism 9 and superimposed onto the sharp vision plane of the spectator's eye 3; simultaneously, the sharp vision plane of the spectator's eye 3 and the refracting power of the spectator's eye 3 for the imaging light beam 5 are modified with the help of the static focusing system 10. At the same time, the depth of focus for the virtual 3D contents displayed on the miniature screen 8 by the microprocessor device 4, being a personal computer, is determined so that objects, laid in the virtual scene at their apparent distance from the spectator equal to the current unmodified sharp vision distance of his/her eye 3, are presented as sharp, while the objects laid closer or further away are presented with a corresponding amount of blur.

### Example 3

An apparatus for virtual 3D image presentation comprises a microprocessor device 4 connected to a single eye optical power measurement system 1, which is connected to a single dynamic focusing system 2 that dynamically modifies the optical power of the spectator's eye 3, and the free-standing screen 7 connected to the microprocessor device 4 is always placed the modified sharp vision distance, irrespective of the accommodation of the spectator's eye 3.

### Example 4

An apparatus for virtual 3D image presentation is the same as in Example 3, with the difference that the microprocessor device 4 is connected to two eye optical power measurement systems 1, where the first one is the system measuring the optical power of the spectator's left eye 3, and the other is the system measuring the optical power of the spectator's right eye 3.

### Example 5

An apparatus for virtual 3D image presentation comprises the microprocessor device 4, connected to a single eye optical power measurement system 1, which is connected to a single screen positioning mechanism 9 that positions the miniature screen 8, where the miniature screen 8, connected to the microprocessor device 4, is always set at the modified sharp vision distance, irrespective of the accommodation of the spectator's eye 3. Furthermore, the static focusing system 10 is placed between the miniature screen 8 and the spectator's eye 3.

The system operates in such a way that the spectator's eye 3 sharp vision plane and surface of either the free-standing screen 7 or the miniature screen 8 are brought about superimposing onto each other, and simultaneously, the depth of focus for the generated virtual 3D contents is determined so that objects, laid in the virtual scene at their apparent distance from the spectator equal to the current unmodified sharp vision distance of his/her eye 3, are presented as sharp, while the objects laid closer or further away are presented with a corresponding amount of blur. The superimposing of the spectator's eye 3 sharp vision plane and the surface of the screen 7, 8 is achieved with the help of the dynamic focusing system 2 or the static focusing system 10, or by positioning dynamically the miniature screen 8 at the modified sharp vision distance with the help of screen positioning mechanism 9. The adequate, i.e., consistent with the current unmodified sharp vision distance of the spectator's eye 3, position of the apparent depth of focus in the generated 3D image is achieved by continuous measurement of the eye's 3 optical power with the help of eye optical power measurement system 1.

An apparatus equipped with two eye optical power measurement systems 1, measuring the current refracting power of the dynamic focusing systems of both eyes, can have a form of eyeglasses or goggles, and the microprocessor device 4 generating 3D image can be an external microcomputer or a game console, equipped with a graphical processing unit able to generate 3D image using Depth of Field technique. The apparatus can be also equipped with two miniature screens 8 displaying the generated 3D contents separately for each of spectator's eyes 3, where the miniature screens 8 displaying the 3D contents are dynamically positioned to the sharp vision plane with the help of screen positioning mechanisms 9, individually for each eye.

**List of Symbols in the Drawing**
- 1.: eye optical power continuous measuring system,
- 2.: dynamic focusing system,
- 3.: spectator's eye,
- 4.: microprocessor device,
- 5.: imaging light beam,
- 6.: measurement light beam,
- 7.: free-standing screen,
- 8.: miniature screen,
- 9.: screen positioning mechanism,
- 10.: static focusing system.

## Claims

1. A method of virtual 3D image presentation, wherein the optical power of the spectator's eye (3) is continuously measured with the eye optical power measurement system (1), and, based on the measured optical power, the sharp vision plane of the spectator's eye (3) is determined, and then the sharp vision plane of the spectator's eye (3) is superimposed onto the surface of the screen (7, 8) presenting the virtual 3D contents, and simultaneously, the depth of focus for the virtual 3D contents is determined, such that objects, laid in the virtual scene at their apparent distance from the spectator equal to the current unmodified sharp vision distance of his/her eye, are presented as sharp, while the objects laid closer or further away are presented with blur, where the amount of blur is a non-decreasing function of the absolute difference of the spectator's eye sharp vision distance and the apparent distance to the object in the virtual scene.

2. A method according to claim 1, wherein the sharp vision plane of the spectator's eye (3) is superimposed onto the surface of the screen (7, 8) presenting the virtual 3D contents with the help of the dynamic focusing system (2), which modifies the refracting power of the spectator's eye (3).

3. A method according to claim 1, wherein the sharp vision plane of the spectator's eye (3) is modified with the static focusing system (10), where the surface of the miniature screen (8) is positioned with the help of the screen positioning mechanism (9) and superimposed onto the spectator's eye (3) sharp vision plane.

4. An apparatus for virtual 3D image presentation comprising a screen presenting virtual 3D contents connected to a microprocessor device, wherein the microprocessor device (4) is connected to at least one eye optical power measuring system (1), which is connected to at least one dynamic focusing system (2) that dynamically modifies the optical power of the spectator's eye (3), and the free-standing screen (7), connected to the microprocessor device (4), is always placed at the modified sharp vision distance, irrespective of the spectator's eye (3) accommodation.

5. An apparatus according to claim 4, wherein the microprocessor device (4) is connected to two eye optical power measurement systems (1), where the first one measures the optical power of the spectator's left eye (3), and the other measures the optical power of the spectator's right eye (3).

6. An apparatus for virtual 3D image presentation comprising at least one built-in miniature screen presenting virtual 3D contents, connected to a microprocessor device, wherein the microprocessor device (4) is connected to at least one eye optical power measuring system (1), which is connected to at least one screen positioning mechanism (9) that dynamically positions the miniature screen (8), and the miniature screen (8), connected to the microprocessor device (4), is always positioned at the modified sharp vision distance, irrespective of the spectator's eye (3) accommodation, and furthermore, the static focusing system (10) placed between the miniature screen (8) and the spectator's eye (3).

7. An apparatus according to claim 6, wherein the microprocessor device (4) is connected to two eye optical power measurement systems (1), where the first one measures the optical power of the spectator's left eye (3), and the other measures the optical power of the spectator's right eye (3).
